(19) Europäisches Patentamt
European Patent Office
Office européen des brevets

(11) Publication number : **0 265 496 B1**

# (12) EUROPEAN PATENT SPECIFICATION

(45) Date of publication of patent specification : **18.03.92 Bulletin 92/12**

(51) Int. Cl.$^5$ : **C07D 211/26,** G01N 33/53

(21) Application number : **87903148.2**

(22) Date of filing : **21.04.87**

(86) International application number : **PCT/US87/00902**

(87) International publication number : **WO 87/06578 05.11.87 Gazette 87/24**

## (54) Flecainide derivatives and their use in immunoassays.

(30) Priority : **25.04.86 US 856572**

(43) Date of publication of application : **04.05.88 Bulletin 88/18**

(45) Publication of the grant of the patent : **18.03.92 Bulletin 92/12**

(84) Designated Contracting States : **AT BE CH DE FR GB IT LI NL SE**

(56) References cited :
**GB-A- 2 045 760**
**US-A- 3 900 481**
**JOURNAL OF MEDICINAL CHEMISTRY, vol. 20, no. 6, 1977, pages 821-826; E.H. BANITT et al.: "Antiarrhythmics. 2. Synthesis and Antiarrhythmic Activity of N-(Piperidylalkyl)trifluoroethoxybenzamides"**
**CHEMISCHE BERICHTE, Band 118, 1985, Seiten 4616-4619, VCH Verlaggesellschaft mbH, Weinheim, DE; G. BLASCHKE et al.: "Herstellung, optische Reinheit und Konfiguration der Flecainid-Enantiomeren"**

(73) Proprietor : **RIKER LABORATORIES, INC.
Building 225-1S-07 3M Center
St. Paul, MN 55144-1000 (US)**

(72) Inventor : **BANITT, Elden, H.
P.O. Box 33427
St. Paul, MN 55133-3427 (US)**
Inventor : **COUDERT, Gérard
233, rue M. Ravel Cidex 28
F-54710 Ludres (FR)**
Inventor : **GALLACHER, Gérard
31 Willoughby Mews
London (GB)**
Inventor : **WALTERS, Roland, L.
724 Concord Lane
Barrington, IL 60020 (US)**
Inventor : **WELLMAN-BEDNAWSKA, Maria
2, rue de l'Ermitage
F-54600 Villers-les-Nancy (FR)**

(74) Representative : **Baillie, Iain Cameron et al
c/o Ladas & Parry, Altheimer Eck 2
W-8000 München 2 (DE)**

EP 0 265 496 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD

This invention relates to derivatives of flecainide and their use in immunoassays for flecainide.

### BACKGROUND OF THE INVENTION

The compound N-(2-piperidylmethyl)-2,5-bis(2,2,2-trifluoroethoxy)benzamide acetate (generic name: flecainide acetate) is a useful antiarrhythmic which is marketed in many countries including the United States. It is described and claimed in United States Patent 3,900,481. Related compounds described and claimed in said patent include those wherein the piperidyl nitrogen is substituted by a lower alkyl group.

It is desirable to have reliable and efficient methods to monitor for drugs in order to assure that therapeutic dosages are maintained while avoiding dosages which could cause undesirable side effects. An immunoassay for flecainide would be a very desirable method for monitoring flecainide levels.

### SUMMARY OF THE INVENTION

The present invention relates to novel compounds or haptens which are derivatives of flecainide and which are useful as precursors for the synthesis of conjugates with proteins, enzymes and other molecules such as fluorescent compounds. Specifically, this invention relates to the compounds of the formula

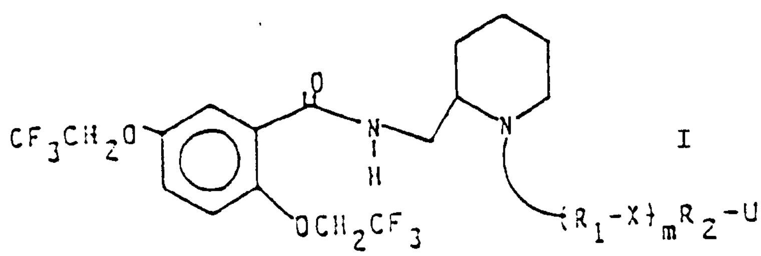

I

wherein:

U is -COOH; -COOR$^8$ wherein R$^8$ is $C_1$ to $C_4$ alkyl; -CHO; -OH; -CN; O

-CNR$^9$NHR$^{10}$, wherein R$^9$ and R$^{10}$ are each independently H or $C_1$ to $C_4$ alkyl; or -NHR$^{11}$, wherein R$^{11}$ is H or $C_1$ to $C_4$ alkyl;

m is 0, 1 or 2;

each X is independently O, S or

$$\overset{R^{12}}{-N-},$$

wherein R$^{12}$ is H or $C_1$ to $C_4$ alkyl;

each $R_1$ is independently alkylene of 2 to 6 carbon atoms, any $R_1$ group further defined as providing an alkylene group of at least 2 carbon atoms which is terminally connected to an separates both i) the piperidyl nitrogen atom and the x moiety closest to the piperidyl nitrogen, and ii) if m is 2, the two X moieties and $R_2$ is alkylene of 1 to 12 carbon atoms, with the provisos that if m is O and U is -OH or -NHR$^{11}$, then $R_2$ has at least 2 carbon atoms in an alkylene group terminally connected to and separating U and the piperidyl nitrogen; if m is 1 or 2 and U is -OH or -NHR$^{11}$, then $R_2$ has at least 2 carbon atoms in an alkylene group terminally connected to and separating U and the X moiety closest to U; and if m is 2, then $R_2$ contains no more than 6 carbon atoms.

Preferred compounds of Formula I are those wherein m is 0 or 1. Most preferred are those wherein m is 0. Any heteroatoms contained as X are preferably oxygen or sulfur.

This invention also relates to compounds of the formula:

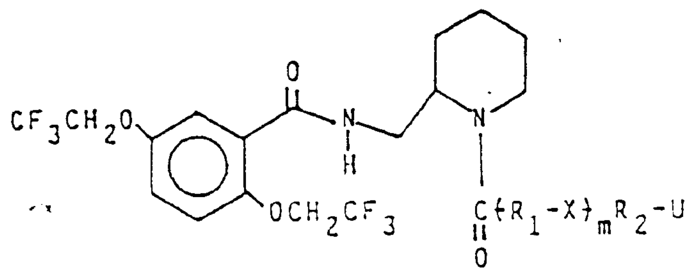

II

wherein:

U is -COOH; -COOR$^8$ wherein R$^8$ is $C_1$ to $C_4$ alkyl; -CHO; -OH;

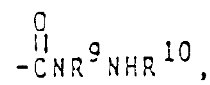

wherein R$^9$ and R$^{10}$ are each independently H or $C_1$ to $C_4$ alkyl; or -NHR$^{11}$, wherein R$^{11}$ is H or $C_1$ to $C_4$ alkyl; m is 0, 1 or 2;

each X is independently O, S or

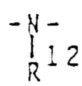

wherein R$^{12}$ is H or $C_1$ to $C_4$ alkyl;

each $R_1$ is independently alkylene of 1 to 6 carbon atoms, with the proviso that if m is 2, then $R_1$ moiety between the two X moieties is an alkylene group of at least two carbon atoms terminally connected to and separating the X moieties; $R_2$ is alkylene of 1 to 12 carbon atoms, optionally containing one or two double bonds, with the provisos that if $R_2$ contains a double bond, then m is 0; if m is 1 or 2 and U is -OH, then $R_2$ has at least 2 carbon atoms in an alkylene group terminally connected to and separating U and the X moiety closest to U; and if m is 2, then $R_2$ contains no more than 6 carbon atoms.

Preferred compounds of Formula II are those wherein m is 0 or 1. Most preferred are those wherein m is 0. Further, preferred are those compounds of Formula II which contain no or only one double bond in $R_2$. Any heteroatoms contained as X are preferably oxygen or sulfur.

This invention also relates to compounds of the formula:

III

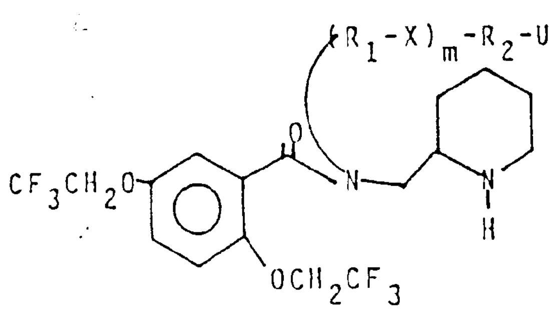

wherein

U is -COOH; -COOR$^8$ wherein R$^8$ is $C_1$ to $C_4$; -CHO; -OH;

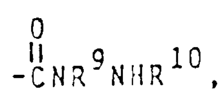

wherein R$^9$ and R$^{10}$ are each independently H or $C_1$ to $C_4$ alkyl; or -NHR$^{11}$, wherein R$^{11}$ is H or $C_1$ to $C_4$ alkyl;

m is 0, 1 or 2;

each X is independently O, S or

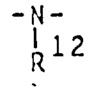

wherein $R^{12}$ is H or $C_1$ to $C_4$ alkyl;

each $R_1$ is independently alkylene of 1 to 6 carbon atoms, with the proviso that if m is 2, then the $R_1$ moiety between the two X moieties is an alkylene group of at least 2 carbon atoms terminally connected to and separating the X moieties and $R_2$ is alkylene of 1 to 12 carbon atoms, with the provisos that if m is 0 and U is -OH or -$NHR^{11}$, then $R_2$ is an alkylene group of at least 2 carbon atoms connected terminally to and separating U and the amide nitrogen; if m is 1 or 2 and U is -OH or -$NHR^{11}$, then $R_2$ is an alkylene group of at least 2 carbon atoms connected terminally to and separating U and the X moiety closest to U; and if m is 2, then $R_2$ contains no more than 6 carbon atoms.

Preferred compounds of Formula III are those wherein m is 0 or 1. Most preferred are those wherein m is 0. Any heteroatoms contained as X are preferably oxygen or sulfur.

In another aspect, the present invention relates to conjugates of certain of the haptens of formulas I, II and III with suitable carriers (e.g., proteins, polysaccharides or other polymeric materials) to provide antigenic materials (or " immunogens").

In still another aspect, the present invention relates to antibody raised in response to the above immunogens.

In still another aspect, the present invention relates to immunoassays for flecainide.

The haptens, immunogens, antibodies and tracers of the present invention are useful in providing reliable immunoassays for flecainide. A variety of types of immunoassays may be conducted using such materials such as fluorescent polarized immunoassay (FPIA) and ELISA (enzyme linked immunosorbent assay).

## DETAILED DESCRIPTION

Alkyl and alkylene groups of the compounds of the invention may be straight or branched chain. By "lower alkyl" is meant alkyl groups containing one to four carbon atoms.

The synthesis of novel compounds of the invention of Formula I wherein U is carboxylate is carried out by the reaction of flecainide with a halogen-substituted alkanoic acid ester. Preferably, the halogen is bromine or iodine and its substitution position is on the carbon farthest from the carbonyl group. Suitable reactants include ethyl 5-bromopentanoate, ethyl alpha-bromoacetate, ethyl 4-bromobutanoate, ethyl 3-bromopropionate, ethyl alpha-iodoacetate, ethyl 6-(2-bromoethoxy)hexanoate, methyl (3-bromopropylthio)acetate, and ethyl 8-bromo-3,6-dioxaoctanoate. The reaction is generally carried out in the presence of a base such as a carbonate or bicarbonate, for example, potassium or sodium bicarbonate, or potassium or sodium carbonate or a hydroxide such as potassium or sodium hydroxide. Generally, the reaction is conducted in an inert, but highly polarizing solvent such as N,N-dimethylformamide or dimethyl sulfoxide at an elevated temperature, for example, 80° to 150°C.

The synthesis of compounds of Formula I wherein U is amino may be carried out by reacting flecainide with, for example, acrylonitrile or a bromo-substituted alkyl nitrile such as 3-(2-bromoethoxy)propionitrile, and thereafter reducing the cyano group, as described hereinafter. Alternatively, such compounds may be prepared by the reaction of flecainide with a (halogen-substituted-alkyl)amine, preferably omega-bromo or omega-iodo-substituted, where the amino group is protected by a blocking group such as carbobenzoxy, followed by deblocking. Another synthetic variation which is useful is to prepare the flecainide precursor wherein the piperidine ring is unreduced, i.e., it is a pyridine ring. The pyridine nitrogen is then reacted with an amino-protected (halogen-substituted-alkyl)amine to form a novel quarternary pyridino nitrogen compound of the formula

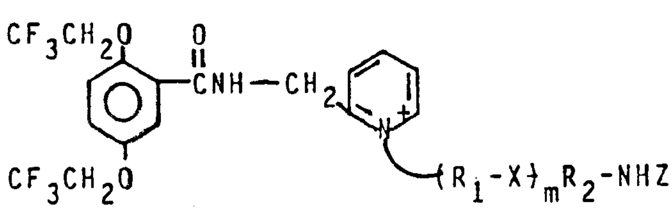

wherein $R_1$, X, m and $R_2$ are as defined above in connection with Formula I, and Z is a blocking group. Subsequently, the pyridino ring is reduced by conventional methods such as one of those described in U. S. Patent 3,900,481. Finally, the blocking group is removed to provide the desired compounds of Formula I wherein Y is amino.

Compounds of Formula I wherein U is monoalkyl amino may be prepared by reductive amination of compounds of Formula I wherein U is -CHO.

Compounds of Formula I wherein U is -OH may be prepared by reacting flecainide with a halogen-substituted primary alkanol such as 3-bromopropanol or 2-(3-bromopropoxy)ethanol.

Compounds of Formula I wherein U is -CHO may be prepared by oxidizing the corresponding compounds wherein U is -OH. Alternatively, such compounds may be prepared by reacting flecainide with a bromoalkylacetal, followed by hydrolysis of the acetal by reaction with acid.

Compounds of Formula I wherein m is 1 or 2 and an X is a secondary amino or tertiary amino may be prepared by stepwise building of the chain using known reagents and methods.

The synthesis of the novel compounds of the invention of Formula II wherein U is carboxylate is carried out by reaction of flecainide with either an anhydride such as succinic anhydride, glutaric anhydride, diglycolic anhydride and the like in an organic base solvent such as pyridine, or with an acyl halide containing a terminal functional group such as 4-(methoxycarbonyl)butyryl chloride, 5-(ethoxycarbonyl)pentanoyl chloride, ethyl malonyl chloride, 4-(4-ethoxycarbonylbutylthio)butyryl chloride, 8-ethoxycarbonyl-4,7-dioxaoctanoyl chloride and the like in the presence of a base.

The synthesis of compounds of Formula II wherein U is amino may be carried out by blocking the amino group of an amino acid, preparing the corresponding acid chloride and reacting the acid chloride with the piperidino nitrogen of flecainide, followed by deblocking the amino group. Suitable blocking groups include the carbobenzoxy group, tertiarybutoxycarbonyl and trifluoroacetyl. Alternatively, the blocked amino acid may be reacted with the piperidino nitrogen of flecainide by any of many peptide synthetic methods, such as by reacting the acid group of the amino acid in the presence of dicyclohexylcarbodiimide. Still alternatively, flecainide may be reacted with a cyano-substituted acetyl chloride such as (2-cyanoethoxy)acetyl chloride to provide a nitrile which can then be reduced to an amine using conventional methods.

Compounds of Formula II wherein U is -CHO may be prepared by reacting flecainide with omega-formyl alkanoic acids such as 4-formylbutyryl chloride or (3-formylpropoxy)acetyl chloride as will be known to one skilled in the art.

Compounds of Formula II wherein U is monoalkylamino may be prepared by reductive amination of compounds of Formula II wherein U is -CHO.

Compounds of Formula II wherein U is -OH may be prepared by reducing corresponding compounds wherein U is -CHO.

Compounds of Formula I wherein m is 1 or 2 and an X is secondary amino or tertiary amino may be prepared by stepwise building of the chain using known reagents and methods.

Compounds of Formula III of the invention wherein U is carboxy may be prepared by reacting flecainide with an omega halogen-substituted alkanoic acid ester in the presence of sodium hydride, followed by saponification of the ester. Suitable esters which may be used include those used to prepare compounds of Formula I wherein U is carboxylate.

Compounds of Formula III wherein U is amino may be prepared by reacting flecainide with a bromo-substituted alkylnitrile such as 3-bromopropionitrile or 3-(2-bromomethoxy)propionitrile, followed by reduction of the nitrile moiety to amino as has been described above.

Compounds of Formula III wherein U is -CHO may be prepared by reacting flecainide with a bromoalkylacetal. Reduction of the aldehyde using conventional methods would then provide the corresponding compound wherein U is -OH. Alternatively, such compounds may be preparred by methods analogous to those for preparing compounds of Formula I wherein U is -OH.

Compounds of Formula III wherein m is 1 or 2 and an X is secondary amino or tertiary amino may be prepared by stepwise building of the chain using known reagents and methods.

Compounds of Formulas I, II, and III wherein U is carboxy may be converted to an activated ester by any conventional method, for example, by reaction with N-hydroxysuccinimide. The activated ester may then be reacted with an amino group of an antigenic protein such as keyhole limpet haemocyanin or bovine serum albumin to provide an immunogen which, when administered to mammals, results in the production of flecainide antibody or antisera. Such activated esters may also be reacted with other antigenic polymers such as polysaccharides as well. Suitable antigenic polymers will be known to one skilled in the art.

Compound of Formulas I, II and III wherein U is $COOR^8$ -CHO, -OH, $-NHR^{11}$ or

$$-\overset{O}{\overset{\|}{C}}NR^9HR^{10}$$

may also be reacted with antigenic polymers such as proteins or polysaccharides to provide immunogens. Suitable chemical methods for providing such immunogens will be known to those skilled in the art.

Further, various compounds of Formulas I, II and III may be reacted with enzymes such as horseradish peroxidase or with fluorescent compounds such as fluorescein or a derivative thereof to provide tracers for conducting an ELISA or fluorescence immunoassay (i.e., FPIA or non-polarized immunoassay), respectively.

It should be noted that the nature of the linking group which is connected to the piperidyl nitrogen atom or amide nitrogen, as the case may be, in the compounds of Formulas I, II and III is not believed to be critical to the performance of such compounds as components of immunogens or tracers since no strongly immunogenic groups are present in the linking group.

A preferred immunoassay for flecainide acetate is an FPIA using the reagents described below, with all percentages being expressed as weight to volume unless otherwise indicated. The tracer formulation presently preferred is 82 nanomolar of the tracer of Example 37 in: 0.1 molar tris buffer at pH 5.5; 5% 5-sulfosalicylate; and 0.1% sodium azide. The antiserum formulation comprises sheep antiserum obtained using the immunogen of Example 24 and diluted with: 1% normal sheep serum (volume/volume); and 0.1% sodium azide. The dilution buffer comprises: 0.1 molar sodium phosphate at pH 7.5; 0.1% sodium azide; and 0.01% bovine gamma globulin. The pretreatment formulation comprises: 0.1 molar tris buffer at pH 5.5; 5% 5-sulfosalicylate; and 0.1% sodium azide. Calibrators comprising flecainide acetate in normal human serum at concentrations of 0.0, 0.1, 0.25, 0.50, 1.0 and 1.5 milligrams per liter, with 0.1% sodium azide preservative are useful. Controls comprising flecainide acetate in normal human serum are provided at concentrations of 0.15, 0.60 and 1.20 milligrams per liter with 0.1% sodium azide as a preservative.

The preferred procedure is especially designed to be used in conjunction with the Abbott TDx polarization analyzer available from Abbott Laboratories, Irving, Texas. In the assay, 50 microliters of serum or plasma are required. The calibrators, controls or unknown samples are pipetted directly into the sample well of the TDx sample cartridge. One of the advantages of this procedure is that the sample does not require any special preparation. If a TDx flecainide acetate assay kit is being used with the TDx analyzer, samples are placed directly into a sample carousel, the caps from each of the three reagent containers in the kit are removed and placed into designated wells inside the TDx analyzer, and the assay procedure from this point is fully automated.

If a manual assay is being performed, the sample is mixed with the pretreatment solution in dilution buffer and a background reading is taken. The tracer is then mixed with the assay. The antibody is then finally mixed into the test solution. After incubation, a fluorescence polarization reading is taken.

The fluorescence polarization value of each calibrator, control or sample is determined and may be printed on the output tape of an instrument such as the Abbott TDx polarization analyzer. A standard curve is generated in the instrument by plotting the polarization of each calibrator versus its concentration using nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored betwen about 2 and 8°C., while the dilution buffer should be stored at ambient temperature. A standard curve and controls should be run every two weeks, with each calibrator and control run in duplicate. Controls should be run daily and all samples can be run in replicates if so desired.

The examples described herein are used to illustrate the invention and are not intended to limit the invention. All amounts given are parts by weight unless otherwise indicated.

EXAMPLE 1 -

Part A

To a solution of 6.2g (0.015 mole) of N-(2-piperidylmethyl)-2,5-bis(2,2,2,-trifluoroethoxy)benzamide in 15 ml of N,N-dimethylformamide was added 3.45g (0.0165 mole) of ethyl 5-bromopentanoate and 1.5g (0.018 mole) of sodium bicarbonate and the mixture was stirred at 120°C. for three hours. After standing for 16 hours, the mixture was diluted with 300 ml of ethyl acetate. This solution was washed sequentially with dilute sodium bicarbonate solution, saturated sodium bicarbonate solution and twice with saturated sodium chloride solution. Drying and evaporation provided a clear, yellow liquid. The liquid was purified by flash chromatography on silica gel, eluting sequentially with chloroform and then 2% (by volume) methanol in chloroform. Evaporation provided clear, yellow liquid N-([1-(4-ethoxycarbonyl)butyl-2-piperidyl]methyl)-2,5-bis(2,2,2-trifluoroethoxy)benzamide. The product was pure according to thin layer chromatography (one spot, $R_f$=0.30) and the structural assignment was supported by infrared and nuclear magnetic resonance spectral analyses.

Part B

To a stirred solution of 6.7g (0.01235 mmole) of N-([1-(4-ethoxycarbonyl)butyl-2-piperidyl]methyl)-2,5-bis(2,2,2-trifluoroethoxy)benzamide in 40 ml of ethanol was added a solution of 0.62g (15.4 mmole) of sodium hydroxide in 40 ml of water. After stirring at 20°C. for about 17 hours, the solution was evaporated to about 50 ml. The solution was washed with ethyl acetate, and the aqueous layer was neutralized to pH 6 by the addition of 3N hydrochloric acid and then cooled to 0°C. A yellow oil separated and was extracted four times with 60 ml portions of ethyl acetate and the ethyl acetate extracts were dried, filtered and evaporated to provide a syrup which was pure N-([1-(4-carboxy)butyl-2-piperidyl]methyl)-2,5-bis(2,2,2-trifluoroethoxy)benzamide according to thin layer chromatography analysis on silica using 10% (by volume) methanol in chloroform ($R_f$=0.09) and 20% (by volume) methanol in chloroform ($R_f$=0.22).

Using the method of Example 1, Part A, flecainide was reacted with the following bromoalkanoic esters to provide the products indicated. In each example, $R_f$ was determined by thin layer chromatography on silica gel, eluting with 20% methanol in chloroform.

TABLE

| Example # | Bromoalkanoic Ester | Product | Physical Properties |
|---|---|---|---|
| 2 | ethyl 4-bromobutanoate | N-([1-(3-ethoxycarbonyl)-propyl-2-piperidyl]-methyl)-2,5-bis-(2,2,2-trifluoroethoxy)-benzamide | Yellow Liquid ($R_f$ = 0.37) |
| 3 | ethyl 3-bromopropanoate | N-([1-(2-ethoxycarbonyl)-ethyl-2-piperidyl]-methyl)-2,5-bis(2,2,2-trifluoroethoxy)-benzamide | Ivory Solid ($R_f$ = 0.46) |
| 4 | ethyl 2-bromoacetate | N-([1-ethoxycarbonyl)-methyl-2-piperidyl]methyl)-2,5-bis(2,2,2-trifluoroethoxy)-benzamide | Yellow Liquid ($R_f$ = 0.66) |

EXAMPLE 5 -

To a solution of 9.4g (18.3 mmole) of N-([1-(2-ethoxycarbonyl)ethyl-2-piperidyl]methyl)-2,5-bis(2,2,2-trifluoroethoxy)benzamide in 60 ml of ethanol was added a solution of 0.91g (22.8 mmole) of sodium hydroxide in 60 ml of water. After stirring for about 48 hours at about 20°C., the solution was concentrated on a rotary evaporator at a temperature no greater than 50°C. The total volume was adjusted to 60 ml by the addition of water, and the solution was then washed with ethyl acetate. The aqueous layer was neutralized to pH 6 by the careful addition of 3N hydrochloric acid. The mixture was cooled, then extracted with four sixty ml portions of ethyl acetate. The ethyl acetate extracts were dried over magnesium sulfate, filtered and then evaporated to dryness to provide glassy amorphous ivory solid N-([1-(2-carboxyl)ethyl-2-piperidyl]methyl)-2,5-bis-(2,2,2-trifluoroethoxy)benzamide. Thin layer chromatographic analysis on silica gel, eluting with 20% (by volume) methanol in chloroform, showed a single spot at $R_f$ = 0.23.

EXAMPLE 6 -

Using the method of Example 5, the ester of Example 2 was hydrolyzed to provide N-([1-(3-carboxy)propyl-2-piperidyl]methyl)-2,5-bis(2,2,2-trifluoroethoxy)benzamide. Thin layer chromatographic analysis on silica gel, eluting with 20% methanol in chloroform, showed a single spot at $R_f$ = 0.33.

EXAMPLE 7 -

Using the method of Example 5, the ester of Example 4 was hydrol-yzed to provide N-[(1-carboxymethyl-2-piperidyl)methyl]-2,5-bis-(2,2,2-trifluoroethoxy)benzamide as an ivory powder. Recrystallization from 67% aqueous ethanol provided white powder, m.p. 224-226°C. Analysis: Calculated for $C_{19}H_{22}F_6N_2O_5$ = %C, 48.3; %H, 4.7; %N, 5.9; Found: %C, 48.1; %H, 4.5, %N, 5.8.

EXAMPLE 8 -

Small portions of 3.0g (7.24 mmole) of N-(2-piperidylmethyl)-2,5-bis(2,2,2-trifluoroethoxy)benzamide were added to 15 ml of stirred acrylonitrile. The solution was heated at reflux for about 16 hours, then evaporated to dryness to provide a syrup which solidified on standing. The crude product was purified by flash chromatography on silica gel, eluting sequentially with chloroform and 2% (by volume) methanol in chloroform, providing white solid N-([1-(2-cyanoethyl)-2-piperidyl]methyl)-2,5-bis-(2,2,2-trifluoroethoxy)benzamide. Recrystallization from ethanol and crystallization below 10°C. provided product which had a melting point of 85-87°C. Analysis: Calculated for $C_{20}H_{23}F_6N_3O_3$:%C, 51.4; %H, 5.0, %N, 9.0; Found: %C, 51.1; %H, 4.8; %N, 8.8.

A solution of 15.0g (32.1 mmole) of N-([1-(2-cyanoethyl)-2-piperidyl]methyl)-2,5-bis(2,2,2-trifluoroethoxy) benzamide in 250 ml of warm ethanol was cooled with an ice bath and saturated with dry ammonia gas. This solution was placed in a hydrogenation vessel (under nitrogen gas) containing about 0.5g of Raney nickel in an ethanol paste, and was hydrogenated for 16 hours on a Paar apparatus at about 20°C and 50 psi. The mixture was filtered, then evaporated to provide a syrup which gradually hardened. Recrystallization from a heptane-toluene mixture and subsequent cooling provided off-white solid N-([1-(3-aminopropyl)-2-piperidyl]methyl)-2,5-bis(2,2,2-trifluoroethoxy)benzamide, m.p. 52- 54°C. Analysis: Calculated for $C_{20}H_{27}F_6N_3O_3$:%C, 51.0; %H, 5.8; %N, 8.9; Found: %C, 51.1; %H, 5.6; %N, 8.8.

EXAMPLE 9 -

Monoethyl adipate (0.6 mmole), 0.9 mmole of o-nitrophenylthiocyanate and 0.9 mmole of N-(2-piperidylmethyl)-2,5-bis(2,2,2-trifluoroethoxy)benzamide were dissolved in 10 ml of tetrahydrofuran which contained 0.9 mmole of tri-n-butylphosphine. The solution was agitated under argon at ambient temperature. The solvent was then evaporated and N-([1-(5-ethoxycarbonyl-1-oxo)pentyl-2-piperidyl]methyl)-2,5-bis(2,2,2-trifluoroethoxy)benzamide obtained was purified using a silica column to provide product in 77% yield.

The N-([1-(5-ethoxycarbonyl-1-oxo)pentyl-2-piperidyl]methyl)-2.5-bis(2,2,2-trifluoroethoxy)benzamide was dissolved in dioxane to which 0.2N sodium hydroxide, 10 ml, was then added. The mixture was agitated and maintained at 50°C. After three hours, the mixture was made acidic and extracted with chloroform (3 x 20 ml). The solvent was evaporated under vacuum. The yield was 85%.

EXAMPLE 10 -

N-(2-piperidylmethyl)-2,5-bis(2,2,2-trifluoroethoxy)benzamide was reacted with monoethyl fumarate using the method of Example 9 to provide N-([1-((3-carbonyl-1-oxo)propen-2-yl)-2-piperidyl]methyl)-2,5-bis-(2,2,2-trifluoroethoxy)benzamide as confirmed by nuclear magnetic resonance spectroscopy. The yield was 84%.

EXAMPLE 11 -

To a stirred solution of 6.2g (0.015 mole) of N-(2-piperidylmethyl)-2,5-bis(2,2,2-trifluoroethoxy)benzamide and 6.4g (0.060 mole) of sodium carbonate in 60 ml of benzene at 20°C. was added 3.5g (0.018 mole) of 5-(ethoxycarbonyl)pentanoyl chloride. Stirring was continued for about 16 hours, and the solution was then evaporated. Water and dichloromethane were added to the residue and the layers were separated. The dichloromethane layer was washed sequentially with dilute hydrochloric acid and saturated sodium chloride solution, and was dried over magnesium sulfate. Filtration provided a solution which was evaporated to provide a colorless syrup. The syrup was dissolved in chloroform and purified by flash chromatography on a silica column to again provide a syrup. Thin layer chromatographic analysis revealed pure N-([1-(5-ethoxycarbonyl-1-oxo)pentyl-2-piperidyl]methyl)-2,5-bis(2,2,2-trifluoroethoxy)benzamide as evidenced by a single spot ($R_f$=0.68) using 10% (by volume) methanol in chloroform as the eluent.

EXAMPLE 12 -

Using the method of Example 11, flecainide was reacted with ethyl malonyl. chloride to provide N-([1-(2-ethoxycarbonyl-1-oxo)ethyl-2-piperidyl]methyl)-2,5-bis(2,2,2-trifluoroethoxy)benzamide. Recrystallization from ethanol provided white solid, m.p. 138-139°C. Analysis: Calculated for $C_{22}H_{26}F_6N_2O_6$:%C, 50.0; %H, 5.0; %N, 5.3; Found: %C, 50.0; %H, 5.0; %N, 5.3.

EXAMPLE 13 -

Using the method of Example 11, flecainide was reacted with 4-(methoxycarbonyl)butyryl chloride to provide crude N-([1-(4-methoxycarbonyl-1-oxo)butyl-2-piperidyl]methyl)-2, 5-bis(2,2,2-trifluoroethoxy)benzamide. Thin layer chromatographic analysis on silica gel, eluting with 5% (by volume) methanol in chloroform, showed two spots at $R_f$ = 0.40 and $R_f$ = 0.22, indicating the product was contaminated with a by-product.

EXAMPLE 14 -

To a stirred solution of 8.4g (0.0147 mole) of N-([1-(5-ethoxycarbonyl-1-oxo)pentyl-2-piperidyl]methyl)-2,5-bis(2,2,2-trifluoroethoxy)benzamide in 60 ml of ethanol was added 0.74g (0.0184 mole) of sodium hydroxide in 60 ml of water. After stirring for about 16 hours, the solution was concentrated by evaporation at less than 50°C. The solution was washed with diethyl ether. The aqueous phase was then acidified with dilute hydrochloric acid, and extracted twice with ether and then with saturated sodium chloride solution. The aqueous phase was then dried over magnesium sulfate, filtered and evaporated to provide a syrup which was dissolved in chloroform and flash chromatographed on florisil to provide N-([1-(5-carboxy-1-oxo)pentyl-2-piperidyl]methyl)-2,5-bis(2,2,2-trifluoroethoxy)benzamide. Thin layer chromatographic analysis on silica gel, eluting with 10% (by volume) methanol in chloroform, showed a single spot at $R_f$ = 0.34.

EXAMPLE 15 -

To a stirred solution of 4.14g (0.010 mole) of N-(2-piperidylmethyl)-2,5-bis(2,2,2-trifluoroethoxy)benzamide in 10 ml of pyridine was added 2.0g (0.02 mole) of succinic anhydride. After stirring for about 16 hours at about 20°C, about 200 ml of ethyl acetate was added. The solution was washed thrice with 100 ml portions of water and then dried. Filtration from the drying agent, magnesium sulfate, followed by evaporation provided white solid which was recrystallized from acetonitrile with treatment with decolorizing charcoal, then recrystallized from ethyl acetate to provide N-([1-(3-carboxy-1-oxo)propyl-2-piperidyl]methyl)-2,5-bis(2,2,2-trifluoroethoxy)benzamide, m.p. 164-165°C. Analysis: Calculated for $C_{21}H_{24}F_6N_2O_6$: %C, 49.0; %H, 4.7; %N, 5.45; Found: %C, 49.1; %H, 4.8; %N, 5.2. The structural assignment was supported by infrared and nuclear magnetic resonance spectral analyses.

EXAMPLE 16 -

Using the method of Example 15, flecainide was reacted with glutaric anhydride to provide N-([1-(4-carboxy-1-oxo)butyl-2-piperidyl]methyl)-2,5-bis(2,2,2-trifluoroethoxy)benzamide. Thin layer chromatographic analysis on silica gel, eluting with 10% (by volume) methanol in chloroform, provided a single spot at $R_f$ = 0.41.

EXAMPLE 17 -

To a stirred solution of 12.4g (0.030 mole) of flecainide and 8.75g (0.033 mole) of N-carbobenzoxy-6-aminohexanoic acid in 100 ml of dichloromethane was added dropwise, over one hour, a solution of 6.2g (0.030 mole) of dicyclohexylcarbodiimide. After stirring for about four hours, the mixture was filtered to remove the solid present and the solid was then washed with dichloromethane with the washing being added to the filtrate. Evaporation of the filtrate provided a residual syrup which was mostly dissolved by 400 ml of diethyl ether and the mixture was then filtered. A saturated hydrogen chloride in diethyl ether solution was added to the filtrate until the pH was acidic. The resulting solid was removed by filtration and the filtrate was evaporated. The residue was the desired product, N-([1-(6-carbobenzoxyamino-1-oxo)hexyl-2-piperidyl]methyl)-2,5-bis-(2,2,2-trifluoroethoxy)benzamide. Thin layer chromatography analysis on silica gel, eluting with 10% (by volume) methanol in chloroform, provided a single spot at $R_f$=0.70.

EXAMPLE 18 -

To a stirred solution of 21.2g (0.051 mole) of flecainide in 200 ml of glyme was added dropwise (over 30 minutes) 7.1g (0.025 mole) of N-carbobenzoxy-6-aminohexanoyl chloride in 40 ml of glyme. After stirring for about 16 hours, the mixture was heated at 65°C. for one hour, cooled, diluted with 200 ml diethyl ether, and filtered to remove the solid present. The solid was washed with diethyl ether, the washings combined with the filtrate, and the filtrate evaporated to dryness. The residual syrup was mostly dissolved by 400 ml of diethyl ether, and the mixture was washed sequentially with dilute hydrochloric acid, dilute sodium carbonate solution and brine, and was then dried. Evaporation provided a syrup which was flash chromatographed over silica gel, eluting sequentially with chloroform, and 2% (by volume) methanol in chloroform to provide a clear, thick syrup which slowly hardened. Recrystallization from 2:1 toluene: heptane with treatment with decolorizing charcoal provided as a white powder N-([1-(6-carbobenzoxyamino-1-oxo)hexyl-2-piperidyl]methyl)-2,5-bis(2,2,2-trifluoroethoxy)benzamide, m.p. 67-69°C. Analysis: Calculated for $C_{31}H_{37}F_6N_3O_6$: %C, 56.3; %H, 5.6; %N, 6.35; Found: %C, 57.0; %H, 6.0; %N, 6.0.

EXAMPLE 19 -

Under nitrogen gas, a solution of 9.9g (0.015 mole) of N-([1-(6-carbobenzoxyamino-1-oxo)hexyl-2-piperidyl]methyl)-2,5-bis(2,2,2-trifluoroethoxy)benzamide in 5 ml of acetic acid and 200 ml of ethanol was added to a hydrogenation vessel which contained a paste prepared by combining 1.0g of 5% palladium on carbon with ethanol. Hydrogenation was carried out on a Paar apparatus for about 16 hours at about 20°C. The mixture was filtered to remove the catalyst and refiltered with added celite. The solution obtained was evaporated to remove ethanol to provide a clear syrup. To the syrup was added 150 ml of water, and the pH of the solution obtained was about 4. Celite was added to the solution and the solution was filtered and basified by slow addition of 10% sodium hydroxide solution. A syrup separated and was extracted twice with diethyl ether. The ether extracts were washed with saturated sodium chloride solution, dried over magnesium sulfate and filtered. The filtrate was evaporated to provide clear colorless syrup of N-([1-(6-amino-1-oxo)hexyl-2-piperidyl]methyl)-2,5-bis(2,2,2-trifluoroethoxy)benzamide. Thin layer chromatographic analysis on silica gel, eluting with 20% (by volume) methanol in chloroform, showed a spot at $R_f$ = 0.11 with a small shoulder at $R_f$ = 0.16.

EXAMPLE 20 -

Using the method of Example 16, flecainide was reacted with N-carbobenzoxy-3-aminopropionyl chloride to provide N-([1-(3-carbobenzoxyamino-1-oxo)propyl-2-piperidyl]methyl)-2,5-bis(2,2,2-trifluoroethoxy)benzamide as a white powder which, after recrystallization from 2:1 toluene:hexane, had a m.p. of 73-76°C. Analysis: Calculated for $C_{28}H_{31}F_6N_3O_6$: %C, 54.3; %H, 5.0; %N, 6.8; Found: %C, 54.0; %H, 5.0; %N, 6.7.

EXAMPLE 21 -

Using the method of Example 19, N-([1-(3-carbobenzoxyamino-1-oxo)propyl-2-piperidyl]methyl)-2,5-bis(2,2,2-trifluoroethoxy)benzamide (from Example 18) was reduced to provide clear, colorless syrup which gradually hardened to white solid N-([1-(3-amino-1-oxo)propyl-2-piperidyl]methyl)-2,5-bis-(2,2,2-trifluoroethoxy)benzamide. Analysis: Calculated for $C_{20}H_{25}F_6N_3O_4$: %C, 49.5; %H, 5.2; %N, 8.7; Found: %C, 49.2; %H, 5.1; %N, 8.3.

EXAMPLE 22 -

Using the method of A. Stoffyn et al., Carbohydrate Research, 1972, 22, 251, flecainide was reacted with ethyl 4-bromobutanoate in the presence of sodium hydride using dimethylsulfoxide as a solvent. The N-(3-ethoxycarbonyl)propyl-N-(2-piperidylmethyl)-2,5-bis(2,2,2-trifluoroethoxy)benzamide so obtained was saponified to provide N-(3-carboxypropyl)-N-(2-piperidylmethyl)-2,5-bis(2,2,2-trifluoroethoxy)benzamide (hereinafter referred to as "flecainide-amide-3-carboxypropyl").

EXAMPLE 23 -

Using the method of Example 17, flecainide N-carbobenzoxy-5-aminopentanoic acid, and dicyclohexylcarbodiimide were reacted. Also, using the method of Example 17, the resulting ester was reduced to provide N-([1-(5-amino-1-oxo)pentyl-2-piperidyl]methyl)-2,5-bis(2,2,2-trifluoroethoxy)benzamide. Analysis by thin layer

chromatography on silica gel, eluting with 20% (by volume) methanol in chloroform, showed the product to have an $R_f$ = 0.10.

EXAMPLE 24 -

To a stirred solution of 87mg (0.17mmole) of N-([1-(4-carboxy)butyl-2-piperidyl]methyl)-2,5-bis-(2,2,2-trifluoroethoxy)benzamide (from Example 1) in 5 ml of acetonitrile and 500 microliters of water was added 20 mg (0.17 mmole) of N-hydroxysuccinimide and 32 mg (0.17 mmole) of 1-ethyl-3-(3-dimethylaminopropyl)carbodiimide hydrochloride. The reaction mixture was stirred at about 20°C. for one hour while monitoring it by thin layer chromatographic analysis on silica gel, eluting with 3:1 (v/v) chloroform: methanol, to provide a solution containing the desired N-succinyloxy activated ester. This solution was added dropwise to a stirred solution of 100 mg of keyhole limpet haemocyanin (KLH) (available from Cambridge Bioscience, Cambridge, England) in 10 ml of 0.1M phosphate buffered saline, pH 8, and 2 ml of pyridine. After stirring for about 16 hours at about 20°C., the solution was dialysed against running tap water for three days. The retentate was lyophilised to provide 100 mg of the keyhole limpet haemocyanin conjugate of N-([1-(4-carboxy)butyl-2-piperidyl]methyl)-2,5-bis(2,2,2-trifluoroethoxy)benzamide (hereinafter referred to as "flecainide-4-carboxybutyl-KLH conjugate").

EXAMPLE 25 -

Using the method of Example 24, N-([1-(3-carboxy-1-oxo)propyl-2-piperidyl]methyl)-2,5-bis(2,2,2-trifluoroethoxy)benzamide was reacted to prepare the N-succinyloxy activated ester. The ester was reacted with keyhole limpet haemocyanin following the method of Example 24 to provide the desired "flecainide-hemisuccinate-KLH conjugate".

EXAMPLE 26 -

To a stirred solution of 10 mg (0.02 mmole) of N-([1-(3-carboxy-1-oxo)propyl-2-piperidyl]methyl)-2,5-bis(2,2,2-trifluoroethoxy)benzamide (from Example 15) in 1 ml of acetonitrile and 100 microliters of water was added 2.3 mg (0.02 mmole) of N-hydroxysuccinimide and 3.8 mg (0.02 mmole) of 1-ethyl-3-(3-dimethylaminopropyl)carbodiimide hydrochloride. The reaction mixture was stirred for one hour at about 20°C., monitoring by thin layer chromatographic analysis, to provide the desired N-succinyloxy activated ester. To this solution was added a solution of 9 mg (0.02 mmole) of fluoresceinthiocarbamylethylene diamine (prepared from fluorescein isothiocyanate isomer 1 and ethylenediamine, both from Sigma Chemicals, Poole, Dorset, England, using the method described in M. Pourfarzaneh et al., Clinical Chemistry (1980) 26, 730-733, in 1 ml of acetonitrile containing 100 microliters of water and 100 microliters of triethylamine, and the resulting mixture was stirred for one hour at about 20°C. To this mixture was added 5 ml of 1M aqueous hydrochloric acid. The solid precipitate was separated by filtration, then dissolved in methanol and separated and purified by preparative thin layer chromatography on silica gel, eluting with 5:1 (v/v) chloroform: methanol, to provide the fluorescein conjugate of the N-([1-(3-carboxy-1-oxo)propyl-2-piperidyl]methyl)-2,5-bis-(2,2,2-trifluoroethoxy)benzamide (hereinafter referred to as fluorescein-hemisuccinate-flecainide).

EXAMPLE 27-

Three mature ewes were each immunized in several intramuscular sites with 4 mg of the flecainide-4-carboxybutyl-KLH conjugate (from Example 24) in 4 ml of an emulsion of saline and Freund's complete adjuvant (1:2.5 by volume). Four weeks later, and every subsequent four weeks, the sheep were reimmunized in the same way, except that 2 mg of immunogen per sheep and incomplete Freund's adjuvants were used. Two weeks after the second reimmunization and two weeks after each subsequent reimmunization, the animals were bled. Initially, a 30 ml sample was obtained for evaluation and subsequently 750 ml of blood was taken after each reimmunization. The three sheep all produced highly specific, high titer antibodies and the antiserum obtained from the first bleed of one sheep was used for the immunoassay after coupling as described in Example 28.

EXAMPLE 28 -

Sheep anti-flecainide serum (2 ml) obtained as described in Example 27 was coupled to magnetizable cellulose following the procedures described in Ann. Clin. Biochem. 1983, 20:227-232. The particles were washed three times with assay diluent buffer, resuspended at a concentration of 50 grams per liter and stored at 4°C.

EXAMPLE 29 -

Using the method of Example 27, ewes were immunized with flecainide-hemisuccinate-KLH conjugate (from Example 25) and highly specific, high titer antibodies and antiserum were obtained. The antiflecainide serum was then coupled to magnetizable cellulose using the procedures described in Example 28.

EXAMPLE 30 -

Serum and standard samples were each mixed with fluorescein-hemisuccinate-flecainide (from Example 26) and a suspension of anti-flecainide serum coupled to a magnetizable solid phase which was obtained in Example 28. After a brief incubation at about 20°C, to allow the labelled and unlabelled hapten to compete for the binding sites on the antibodies, the magnetic particles were sedimented on a magnet, and the supernatents were discarded. The bound fractions were released by an eluting reagent consisting of 4 parts methanol to 1 part 0.5M, pH 9, sodium bicarbonate solution (v/v), and the particles were resedimented magnetically. The fluorescence of the eluates was measured directly and the amount of unlabelled drug present was found to be inversely related to the amount measured.

This assay was used on serum sample volumes of 20 microliters. The assay was also used to increase sensitivity by increasing serum sample volume to 500 microliters, using one half as much solid phase anti-serum and one third the concentration of fluorescein-labelled flecainide. As a result, serum levels of one nanogram per ml or less could be accurately detected.

EXAMPLE 31 -

Serum and standard samples (20 microliters) were each added to a solution of fluorescein-hemisuccinate-flecainide (150 nanomoles per liter) followed by 100 microliters of flecainide antibody bonded to magnetizable methyl cellulose solid (obtained as described in Example 29) at a concentration of 4 grams per liter, and the mixture was incubated for one hour. To the stirred mixture was added 1 ml of diluent buffer, sodium borate (200 mmole/liter;pH9) containing 1g/l "Triton X-100" (RIM) and 1g/l sodium azide, and the solid phase was then sedimented on a magnet and the supernatant was discarded. To the solid phase was added 1.4 ml of elution reagent, 4 parts methanol to 1 part 0.5M, pH 9 sodium bicarbonate solution (v/v), and the solide phase was resedimented on the magnet and the fluorescence of the eluted bound fraction was measured using a model LS-20 filter fluorimeter (Perkin-Elmer Corp.). When the results for the standard curve were obtained using flecainide levels ranging from 0 to 100 ng/ml, the sensitivity obtained was at least 1 ng/ml.

EXAMPLE 32 -

Using the method of Example 31, the specificity of the assay was measured by comparing cross reactivity of numerous common pharmaceutical substances, including the two major known metabolites of flecainide, namely the meta-O-dealkylated metabolite and the meta-O-dealkylated lactam of flecainide. When measured at concentrations of 1g/l no cross reactivity was observed.

EXAMPLE 33 -

A single reagent polarization fluoroimmunoassay was run as follows:

The single reagent was prepared as follows: A 5 nanomole per liter solution of fluorescein-hemisuccinate-flecainide (from Example 26) was prepared using a 100 nanomole per liter sodium borate buffer (pH 9.0) containing 1 g per liter of gelatin and 1 g per liter of sodium azide as the diluent. Similarly, a 1:5000 dilution of uncoupled flecainide antiserum obtained in Example 27 was prepared, again using the above buffer as the diluent. One part by volume of the above fluorescein-hemissucinate-flecainide solution was then mixed with 2 parts by volume of the above antiserum solution. Sodium salicytate was added to the above mixture to a final concentration of 1 g per liter.

In conducting the assay, 5 microliters of serum or standard was added to 1500 microliters of the above single reagent, and the mixture was incubated for 1 hour at ambient temperature. The polarization fluorescence was then measured using the fluorimeter described in Example 31.

EXAMPLE 34 -

Immunogens were prepared using the haptens of Examples 9, 10, 15 and 22 as follows:

A solution was prepared by combining 0.07 mmole of one of the above-mentioned haptens and 1.5 ml of N,N-dimethylformamide. To this solution was then added 50 microliters of tri-n-butylamine and 12.5 microliters of isobutylchloroformate. The agitated solution was then placed in an ice bath.

One hundred twenty-five mg of bovine serum albumin or "BSA" (i.e., that available under the trade designation "Pentex Fraction V" from Miles Laboratories) was combined with 2.25 ml of N,N-dimethylformamide, 3 ml of water and 0.125 ml of 1N sodium hydroxide to provide a solution which was then placed in an ice bath.

Thirty minutes later, the above two solutions were combined and maintained at 4°C., and agitated overnight. Then, after dialysis against distilled water for 48 hours, the product was lyophilized to provide the following immunogens:

"flecainide-(5-carboxy-1-oxo)pentyl-BSA" (obtained using the hapten of Example 9);

"flecainide-fumarate-BSA" (obtained using the hapten of Example 10);

"flecainide-(3-carboxy-1-oxo)propyl-BSA" (obtained using N-([1-(3-carboxy-1-oxo)propyl-2-piperidyl]methyl)-2,5-bis(2,2,2-trifluoroethoxy)benzamide (prepared using a procedure similar to that of Example 15);

"flecainide-amide-3-carboxypropyl-BSA" (obtained using the hapten of Example 22).

The immunogens obtained above were then analyzed using the method of B. F. Erlanger et al., J. Biol. Chem. 1957, 228, 713, to determine the number of haptens coupled to each molecule of bovine serum albumin. The results were as follows:

| Immunogens | Number of Flecainide Molecules per Molecule of BSA |
|---|---|
| flecainide-(5-carboxy-1-oxo)pentyl-BSA | 39 |
| flecainide-fumarate-BSA | 14 |
| flecainide-(3-carboxy-1-oxo)propyl-BSA | 19 |
| flecainide-amide-3-carboxypropyl-BSA | 29 |

EXAMPLE 35 -

Tracers were prepared using the haptens of Examples 9, 10, 15 and 22 as follows:

Solution I was prepared by combining 1.1 mg of one of the above-mentioned haptens in 0.5 ml of dioxane. Ten microliters of tri-n-butylamine and 5 microliters of ethylchloroformate were subsequently added to the above solution and the mixture was agitated for about 30 minutes. Solution II was prepared by combining 0.5 ml of a 4 mg/ml aqueous solution of dialyzed peroxidase (i.e., horseradish peroxidase, 250 U per mg of protein, obtained from Boehringer Mannheim) and 5 microliters of tri-n-butylamine.

The above two solutions were combined and left overnight at 4°C. The mixture was then chromatographed over "Sephadex G-25" (available from Pharmacia) and the fraction corresponding to the maximum absorption peak of the immunogen was retained and frozen at -40C.

The tracers obtained using the above method are denoted below:

"flecainide-(5-carboxy-1-oxo)pentyl-peroxidase" (obtained using the hapten of Example 9);

"flecainide-fumarate-peroxidase" (obtained using the hapten of Example 10);

"flecainide-(3-carboxy-1-oxo)propyl-peroxidase" (obtained using N-([1-(3-carboxy-1-oxo)propyl-2-piperidyl]methyl)-2,5-bis(2,2,2-trifluoroethoxy)benzamide prepared using a procedure similar to that of Example 15); and

"flecainide-amide-3-carboxypropyl-peroxidase" (obtained using the hapten of Example 22).

EXAMPLE 36 -

Male rabbits (species: fauves de bourgogne obtained in France) weighing 2.5 - 3 kg were immunized using flecainide-fumarate-BSA, flecainide-(3-carboxy-1-oxo)propyl BSA, flecainide-amide-3-carboxypropyl-BSA, and flecainide-(5-carboxy-1-oxo)pentyl-BSA (prepared in Example 34) as follows:

The rabbits were inoculated subcutaneously at 20 to 30 sites with a solution prepared by mixing 0.5 or 1 mg of an immunogen in 0.5 ml of physiological serum and 0.5 ml of complete Freund adjuvant. Booster injections using incomplete Freund adjuvant were given at intervals of 15 days each. Each rabbit was bled from the

ear vein eight days after the sixth booster challenge.

For the preparation of the immunoglobulin G fraction, whole blood from each of the above was centrifuged and obtained serum was treated with caprylic acid in accordance with the method of M. Steinbuch et al., C. R. Soc. Biol., Paris, 1970, 164, 296.

EXAMPLE 37 -

An ELISA was run using the following general procedure.

One hundred microliters of a 0.1 M, pH 7.2 sodium phosphate buffer solution containing 10 micrograms of the antibody (the immunoglobulin G fraction) prepared in response to the immunogen flecainide-(3-carboxy-1-oxo)propyl-BSA were added to each well of a microtiter plate (e.g., Nunc Microwell Modules) which was then incubated for 16 hours at room temperature. The plate was subsequently rinsed four times with the sodium phosphate buffer, and 120 microliters of a 0.1% BSA solution were added to each well and allowed to incubate for 30 to 60 minutes at room temperature.

Sufficient flecainide-fumarate-peroxidase tracer was dissolved in a 4% solution of BSA in 0.1 M, pH 7.2 sodium phosphate buffer to provide a 1/16000 dilution of the tracer. To each well was then added fifty microliters of the tracer solution together with 50 microliters of standard flecainide solution (in the sodium phosphate buffer) or the sodium phosphate buffer or a plasma sample. The plate was then incubated for 2 hours at room temperature. After rinsing the plate with the sodium phosphate buffer, 100 microliters of a 0.3% solution of o-phenylenediamine dihydrochloride in 0.1 M citrate-phosphate buffer, pH 5.8, containing 0.02% v/v hydrogen peroxide was added to each well. After incubating the plate for thirty minutes in the dark, the enzyme reaction was stopped by adding 100 microliters of 1N sulfuric acid to each well, and the absorbence of the solution was read at 492 nanometers.

By following the above procedure, the limits of detection of flecainide in serum was found to be between 1 and 100 nanograms per well.

Since detergent can adversely affect the hydrophobic bonding of antibody to the microtiter plate, presence of detergents should be avoided.

While in the above study 10 micrograms of antibody was added to each well of the microtiter plate, it was found that about 600 ng is the optimal amount to add. Further, the concentration of BSA in the various solutions used in the above-described procedure may desirably be 1% by weight.

Human serum will preferably be diluted (e.g., a 1 to 50 dilution) before analysis.

EXAMPLE 38 -

A mixture of N-([1-(3-aminopropyl)-2-piperidyl]methyl)-2,5-bis(2,2,2-trifluoroethoxy)benzamide 4.7 mg and 5.3 mg of 2-(fluorescein-5-ylamino)-4,6-dichloro-1,3,5-triazine (Research Organics, Cleveland, Ohio; catalog number 2096D) was stirred at ambient temperature in 0.2 ml of methanol for 24 hours. The product, N-((1-(3-(4-(fluorescein-5-ylamino)-6-chloro-1,3,5-triazine-2-ylamino)propyl)-2-piperidyl)methyl)-2,5-bis(2,2,2-trifluoroethoxy)benzamide was purified by chromatography on silica gel thin layer plates with sequential development using chloroform-methanol and benzene-ethyl acetate-acetone.

EXAMPLE 39 -

Flecainide is preferably prepared in accordance with the procedures described in copending application U.S. S.N. 772,470, filed September 4, 1985.

## Claims

1. A compound of the formula

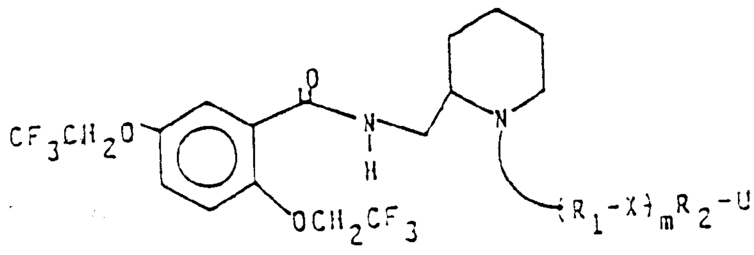

wherein:

U is -COOH; $-COOR^8$ wherein $R^8$ is $C_1$ to $C_4$ alkyl; -CHO; -OH; -CN;

$$-\overset{O}{\overset{\|}{C}}NR^9NHR^{10},$$

wherein $R^9$ and $R^{10}$ are each independently H or $C_1$ to $C_4$ alkyl; or $-NHR^{11}$, wherein $R^{11}$ is H or $C_1$ to $C_4$ alkyl; m is 0, 1 or 2; each X is independently O, S or

$$\overset{R^{12}}{\overset{|}{-N-}},$$

wherein $R^{12}$ is H or $C_1$ to $C_4$ alkyl;

each $R_1$ is independently alkylene of 2 to 6 carbon atoms, any $R_1$ group further defined as providing an alkylene group of at least 2 carbon atoms which is terminally connected to and separates both i) the piperidyl nitrogen atom and the X moiety closest to the piperidyl nitrogen, and ii) if m is 2, the two X moieties and $R_2$ is alkylene of 1 to 12 carbon atoms, with the provisos that if m is O and U is -OH or $-NHR^{11}$, then $R_2$ has at least 2 carbon atoms in an alkylene group terminally connected to and separating U and the piperidyl nitrogen; if m is 1 or 2 and U is -OH or $-NHR^{11}$, then $R_2$ has at least 2 carbon atoms in an alkylene group terminally connected to and separating U and the X moiety closest to U; and if m is 2, then $R_2$ contains no more than 6 carbon atoms.

2. A compound according to Claim 1, wherein U is -COOH.

3. The compound N-([1-(4-carboxybutyl-2-piperidyl]methyl)-2,5-bis-(2,2,2-trifluoroethoxy)benzamide according to Claim 1.

4. A compound according to Claim 1, wherein U is $-NH_2$.

5. The compound N-([1-(3-aminopropyl)-2-piperidyl]methyl)-2,5-bis(2,2,2-trifluoroethoxy)benzamide according to Claim 1.

6. An immunogen comprising a compound according to Claim 1, wherein U is other than -CN bonded via the U moiety to an antigenic polymer.

7. A immunogen comprising a compound according to Claim 2 bonded via the U moiety to an antigenic polymer.

8. An immunogen according to Claim 7, wherein said macromolecule is keyhole limpet haemocyanin.

9. An antibody prepared in response to an immunogen according to Claim 6.

10. An antibody prepared in response to an immunogen according to Claim 7.

11. A compound of the formula

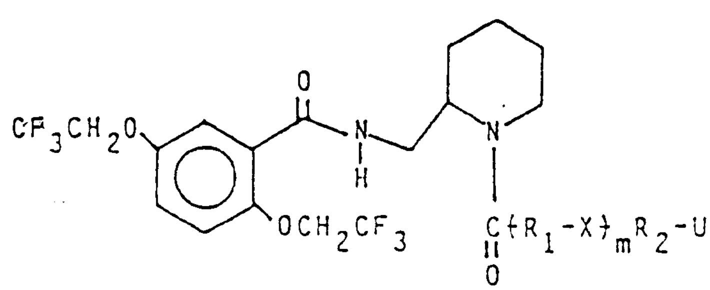

wherein:

U is -COOH; $-COOR^8$ wherein $R^8$ is $C_1$ to $C_4$ alkyl; -CHO; -OH;

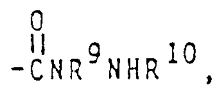

wherein $R^9$ and $R^{10}$ are each independently H or $C_1$ to $C_4$ alkyl; or $-NHR^{11}$, wherein $R^{11}$ is H or $C_1$ to $C_4$ alkyl; m is 0, 1 or 2; each X is independently O, S or

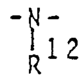

wherein $R^{12}$ is H or $C_1$ to $C_4$ alkyl;

each $R_1$ is independently alkylene of 1 to 6 carbon atoms, with the proviso that if m is 2, then the $R_1$ moiety between the two X moieties is an alkylene group of at least two carbon atoms terminally connected to and separating the X moieties; $R_2$ is alkylene of 1 to 12 carbon atoms, optionally containing one or two double bonds, with the provisos that if $R_2$ contains a double bond, then m is 0; if m is 1 or 2 and U is -OH, then $R_2$ has at least 2 carbon atoms in an alkylene group terminally connected to and separating U and the X moiety closest to U; and if m is 2, then $R_2$ contains no more than 6 carbon atoms.

12. A compound according to Claim 11, wherein U is -COOH.

13. The compound N-([1-(3-carboxy-1-oxo)propyl-2-piperidyl]methyl)-2,5-bis-(2,2,2-trifluoroethoxy)benzamide according to Claim 11.

14. The compound N-([1-(5-carboxy-1-oxo)pentyl-2-piperidyl]methyl)-2,5-bis(2,2,2-trifluoroethoxy)benzamide according to Claim 11.

15. The compound N-([1-((3-carboxy-1-oxo)propen-2-yl)-2-piperidyl]methyl)-2,5-bis(2,2,2-trifluoroethoxy) benzamide according to Claim 11.

16. An immunogen comprising a compound according to Claim 11 bonded via the U moiety to an antigenic polymer.

17. A tracer which is the reaction product of the N-succinyloxy activated ester of N-[1-(3-carboxy-1-oxo)-propyl-2-piperidyl]methyl)-2,5-bis(2,2,2-trifluoroethoxy)benzamide and fluoroesceinthiocarbamylethylene diamine.

18. An antibody prepared in response to an immunogen according to Claim 16.

19. A compound of the formula

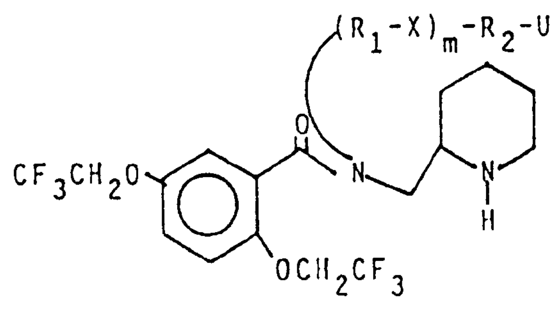

wherein

m is 0, 1 or 2; and $R_1$, U and X are as defined in claim 11. $R_2$ is alkylene of 1 to 12 carbon atoms, with the provisos that if m is O and U -OH or $-NHR^{11}$, then $R_2$ is an alkylene group of at least 2 carbon atoms connected terminally to and separating U and the amide nitrogen; if m is 1 or 2 and U is -OH or $-NHR^{11}$, then $R_2$ is an alkylene group of at least 2 carbon atoms connected terminally to and separating U and the X moiety closest to U; and if m is 2, then $R_2$ contains no more than 6 carbon atoms.

20. A compound according to Claim 19, wherein U is -COOH.

21. The compound N-(3-carboxypropyl)-N-(2-piperidylmethyl)-2,5-bis-(2,2,2-trifluoroethoxy)benzamide according to Claim 19.

22. An immunogen comprising a compound according to Claim 19, wherein U is other than -CN bonded via the U moiety to an antigenic polymer.

23. An antibody raised in response to an immunogen according to Claim 22.

**Patentansprüche**

1. Verbindung der Formel

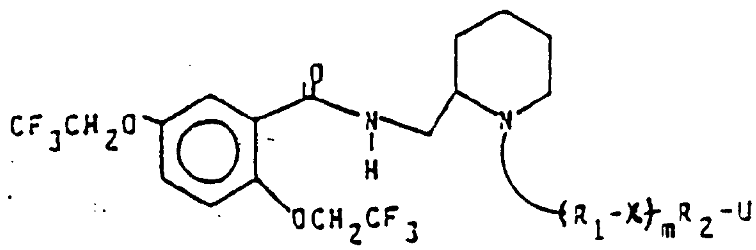

in der

U = -COOH; -$COOR^8$, wobei $R^8$=$C_1$- bis $C_4$-Alkyl ist; -CHO; -OH; -CN; -$CNR^9NHR^{10}$, wobei $R^9$ und $R^{10}$ unabhängig voneinander jeweils H oder $C_1$- bis $C_4$-Alkyl sind; oder -$NHR^{11}$, wobei $R^{11}$=H oder $C_1$- bis $C_4$-Alkyl ist; m = 0, 1 oder 2 ist. jedes X unabhängig von den anderen O, S oder

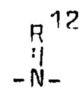

ist, wobei $R^{12}$=H oder $C_1$- bis $C_4$-Alkyl ist;

jedes $R_1$ unabhängig von den anderen ein alkylen mit 2 bis 6 Kohlenstoffatomen ist; jede Gruppe $R_1$ ferner dadurch definiert ist, daß sie eine Alkylengruppe mit mindestens 2 Kohlenstoffatomen besitzt, die i) mit dem Piperidylstickstoffatom und dem dem Piperidylstickstoff nächstliegenden Anteil X endständig verbunden ist und sie voneinander trennt; und $R_2$ ein Alkylen mit 1 bis 12 Kohlenstoffatomen ist, mit der Maßgabe, daß wenn m = O ist und U = -OH oder -$NHR^{11}$ ist, $R_2$ mindestens 2 Kohlenstoffatome in einer Alkylengruppe enthält, die mit U und dem Piperidylstickstoff endständig verbunden ist und sie voneinander trennt; wenn m = 1 der 2 ist und U = -OH oder -$NHR^{11}$ ist, $R_2$ mindestens 2 Kohlenstoffatome in einer Alkylengruppe enthält, die mit U und dem U nächstliegenden Anteil X endständig verbunden ist und sie voneinander trennt; und wenn m = 2 ist $R^2$ höchstens 6 Kohlenstoffatome enthält.

2. Verbindung nach Anspruch 1, dadurch gekennzeichnet, daß U = -COON ist.

3. Verbindung N-([1-(4Carboxybutyl-2-piperidyl]methyl)-2,5-bis-(2,2,2-trifluorethoxy)benzamid nach Anspruch 1.

4. Verbindung nach Anspruch 1, dadurch gekennzeichnet, daß U = -$NH_2$ ist.

5. Verbindung N-([1-(3-Aminopropyl)-2-piperidyl] methyl)-2,5-bis(2,2,2-trifluorethoxy)benzamid nach Anspruch 1.

6. Immunogen mit einer Verbindung nach Anspruch 1, in der U nicht -CN ist und die durch den Anteil U mit einem antigenen Polymer verbunden ist.

7. Immunogen mit einer Verbindung nach Anspruch 2, die durch den Anteil U mit einem antigenen Polymer verbunden ist.

8. Immunogen nach Anspruch 7, dadurch gekennzeichnet, daß das genannte Makromolekül ein schlüssellochartig formangepaßtes Hämocyanin ist.

9. In einer Reaktion auf ein Immunogen nach Anspruch 6 gebildeter Antikörper.

10. In einer Reaktion auf ein Immunogen nach Anspruch 7 gebildeter Antikörper.

11. Verbindung der Formel

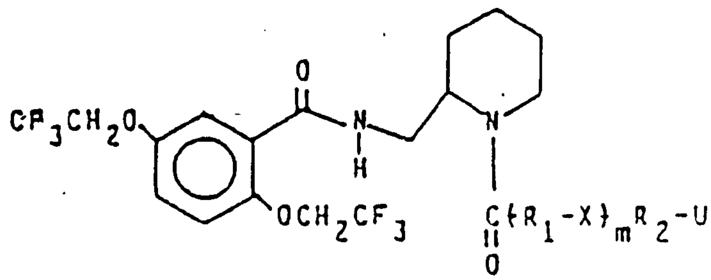

In der

U = -COOH; $-COOR^8$, wobei $R^8=C_1$- bis $C_4$-Alkyl ist; -CHO; -OH; $-CNR^9NHR^{10}$, wobei $R^9$ und $R^{10}$ unabhängig voneinander jeweils H oder $C_1$- bis $C_4$-Alkyl sind; oder $-NHR^{11}$, wobei $R^{11}$=H oder $C_1$- bis $C_4$-Alkyl ist; m = 0, 1 oder ist, jedes X unabhängig von den anderen O, S oder

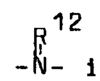

ist, wobei $R^{12}$=H oder $C_1$- bis $C_4$-Alkyl ist;

jedes $R_1$ unabhängig von den anderen ein Alkylen mit 1 bis 6 Kohlenstoffatomen ist, mit der Maßgabe, daß, wenn m = 2 ist der Anteil $R_1$ zwischen den beiden Anteilen X eine Alkylengruppe mit mindestens zwei Kohlenstoffatomen ist, die mit den Anteilen X endständig verbunden ist und sie voneinander trennt; $R_2$ ein Alkylen mit 1 bis 12 Kohlenstoffatomen und gegebenenfalls einer oder zwei Doppelbindungen ist, mit der Maßgabe, daß, wenn $R_2$ eine Doppelbindung ist m = 0 ist; wenn m = 1 oder 2 und U = -OH ist, $R_2$ mindestens zwei Kohlenstoffatome in einer Alkylengruppe besitzt, die mit U und dem U nächstliegenden Anteil X endständig verbunden ist und sie voneinander trennt; und wenn m = 2 ist R höchstens 6 Kohlenstoffatome enthält.

12. Verbindung nach Anspruch 11, dadurch gekennzeichnet, daß Y = -COON ist.

13. Die Verbindung N-([1-(3-carboxy-1-oxo)propyl-2-piperidyl]-methyl)-2,5-bis-(2,2,2-trifluorethoxy)benzamid nach Anspruch 11.

14. Die Verbindung N-([1-(5-carboxy-1-oxo)-pentyl-2-piperidyl]-methyl)-2,5-bis(2,2,2-trifluorethoxy)benzamid nach Anspruch 11.

15. Die Verbindung N-([1-((3-Carboxy-1-oxo)propen-2-yl)-2-piperidyl]methyl)-2,5-bis (2,2,2-trifluorethoxy) benzamid nach Anspruch 11.

16. Immunogen mit einer Verbindung nach Anspruch 11, die durch den Anteil U mit einem antigenen Polymer verbunden ist.

17. Markierungselement, das das Reaktionsprodukt des mit N-Succinyloxy aktivierten Esters von N-([1-((3-carboxy-1-oxo)propen-2-yl)-2-piperidyl] methyl)-2,5-bis(2,2,2-trifluorethoxy)benzamid ist.

18. Durch eine Reaktion auf ein Immunogen nach Anspruch 16 gebildeter Antikörper.

19. Verbindung der Formel

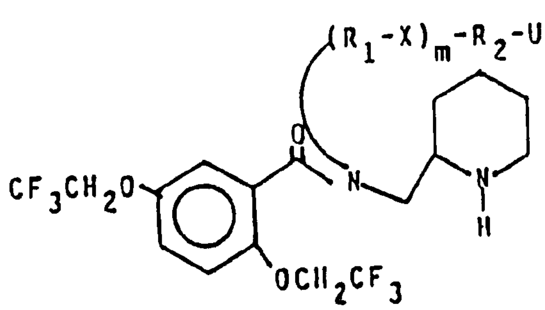

in der

m = 0, 1 oder 2 ist und $R_1$, U und X wie im Anspruch 11 definiert sind, $R_2$ ein Alkylen mit 1 bis 12 Kohlenstoffatomen ist, mit der Maßgabe, daß, wenn m = O und U = $-NHR^{11}$ ist $R_2$ eine Alkylengruppe mit mindestens

2 Kohlenstoffatomen ist, die mit U und dem Amidstickstoff endständig verbunden ist und sie voneinander trennt; wenn m = 1 oder 2 und U = -OH oder -NHR[11] ist $R_2$ eine Alkylengruppe mit mindestens 2 Kohlenstoffatomen ist, die mit U und dem U nächstliegenden Anteil X endständig verbunden ist und sie voneinander trennt; und wenn m = 2 ist $R_2$ höchstens 2 Kohlenstoffatome enthält.

20. Verbindung nach Anspruch 19, dadurch gekennzeichnet, daß U -COOH ist.

21. Die Verbindung N-(3-Carboxypropyl)-N-(2-piperidylmethyl)-2,5-bis-(2,2,2-trifluorethoxy)benzamid nach Anspruch 19.

22. Immunogen mit einer Verbindung nach Anspruch 19, dadurch gekennzeichnet, daß U nicht -CN ist und die durch den Anteil U mit einem antigenen Polymer verbunden ist.

23. Durch eine Reaktion auf ein Immunogen nach Anspruch 22 gebildeter Antikörper.

**Revendications**

1. Composé répondant à la formule :

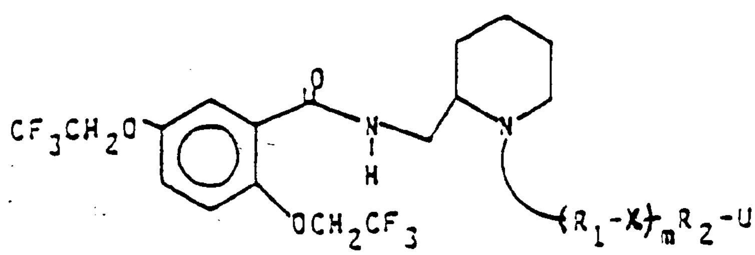

dans laquelle :

U représente -COOH; -COOR⁸, où $R^8$ est un groupe alkyle en $C_1$-$C_4$; -CHO; -OH; -CN;

$$-\overset{O}{\overset{\|}{C}}NR^9NHR^{10},$$

où $R^9$ et $R^{10}$ représentent chacun indépendemment H ou un groupe alkyle en $C_1$-$C_4$; ou -NHR¹¹, où $R^{11}$ représente H ou un groupe alkyle en $C_1$-$C_4$ ; m est égal à 0, 1 ou 2; chaque X représente indépendamment O, S ou

$$\overset{R^{12}}{\overset{|}{-N-}},$$

où $R^{12}$ représente H ou un groupe alkyle en $C_1$-$C_4$;

chaque $R^1$ représente indépendamment un groupe alkylène de 2 à 6 atomes de carbone, tout groupe $R_1$ quelconque étant en outre défini comme donnant un groupe alkylène d'au moins 2 atomes de carbone, qui est relié en bout à et sépare à la fois (i) l'atome d'azote de pipéridyle et le fragment X le plus proche de l'atome d'azote de pipéridyle, et (ii) si m est égal à 2, les deux fragments X, et $R_2$ est un groupe alkylène 1 à 12 atomes de carbone, sous les conditions que, si m est égal à 0 et U représente -OH ou -NHR¹¹, $R_2$ comporte alors au moins deux atomes de carbone dans un groupe alkylène relié en bout à et séparant U et l'azote de pipéridyle; si m est égal à 1 ou 2 et U représente -OH ou -NHR¹¹, $R_2$ comporte alors au moins 2 atomes de carbone dans un groupe alkylène relié en bout à et séparant U et le fragment X le plus proche de U; et si m est égal à 2, $R_2$ ne comporte alors pas plus de 6 atomes de carbone.

2. Composé suivant la revendication 1, dans la formule duquel U représente -COOH.

3. Le composé N-([1-(4-carboxybutyl-2-pipéridyl]méthyl)-2,5-bis-(2,2,2-trifluoréthoxy)benzamide suivant la revendication 1.

4. Composé suivant la revendication 1, dans la formule duquel U représente $-NH_2$.

5. Le composé N-([1-(3-aminopropyl)-2-pipéridyl]méthyl)-2,5-bis(2,2,2-trifluoréthoxy)benzamide suivant la revendication 1.

6. Immunogène comprenant un composé suivant la revendication 1, dans la formule duquel U est différent

de -CN, lié par l'intermédiaire du fragment U à un polymère antigénique.

7. Immunogène comprenant un composé suivant la revendication 2, lié par l'intermédiaire du fragment U à un polymère antigénique.

8. Immunogène suivant la revendication 7, caractérisé en ce que la macromolécule est une hémocyanine (keyhole limpet haemocyanin).

9. Anticorps préparé sous l'influence d'un immunogène suivant la revendication 6.

10. Anticorps préparé sous l'influence d'un immunogène suivant la revendication 7.

11. Composé répondant à la formule:

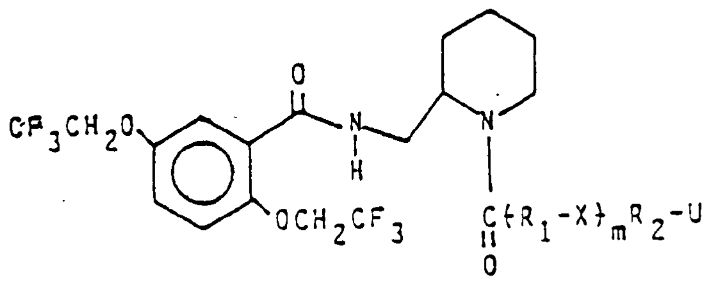

dans laquelle :

U représente -COOH; $-COOR^8$, où $R^8$ est un groupe alkyle en $C_1$-$C_4$; -CHO; -OH;

$$-\overset{O}{\overset{\|}{C}}NR^9NHR^{10},$$

où $R^9$ et $R^{10}$ représentent chacun indépendamment H ou un groupe alkyle en $C_1$ à $C_4$; ou $-NHR^{11}$, où $R^{11}$ représente H ou un groupe alkyle en $C_1$-$C_4$; m est égal à 0, 1 ou 2; chaque X représente indépendamment 0, S ou

$$\underset{R^{12}}{\underset{|}{-N-}},$$

où $R^{12}$ représente H ou un groupe alkyle en $C_1$-$C_4$;

chaque $R_1$ représente indépendamment un groupe alkylène de 1 à 6 atomes de carbone, à la condition que, si m est égal à 2, le fragment $R_1$ compris entre les deux fragments X est alors un groupe alkylène d'au moins deux atomes de carbone, lié en bout à et séparant les fragments X; $R_2$ est un groupe alkylène de 1 à 12 atomes de carbone, contenant facultativement une ou deux doubles liaisons, sous les conditions que, si $R_2$ comporte une double liaison, m est alors égal à 0; si m est égal à 1 ou 2 et U représente -OH, $R_2$ comporte alors au moins deux atomes de carbone dans un groupe alkylène relié en bout à et séparant U et le fragment X le plus proche de U; et si m est égal à 2, $R_2$ ne comporte alors pas plus de 6 atomes de carbone.

12. Composé suivant la revendication 11, dans la formule duquel U représente -COOH.

13. Le composé N-([1-(3-carboxy-1-oxo)propyl-2-pipéridyl]- méthyl)-2,5-bis-(2,2,2-trifluoréthoxy)benzamide suivant la revendication 11.

14. Le composé N-([1-(5-carboxy-1-oxo)pentyl-2-pipéridyl]-méthyl)-2,5-bis(2,2,2-trifluoréthoxy)benzamide suivant la revendication 11.

15. Le composé N-([1-((3-carboxy-1-oxo)propén-2-yl)-2-pipéridyl]méthyl)-2,5-bis(2,2,2-trifluoréthoxy)benzamide suivant la revendication 11.

16. Immunogène comprenant un composé suivant la revendication 11, lié par l'intermédiaire du fragment U à un polymère antigénique.

17. Traceur qui est le produit de réaction de l'ester N-succinyloxy activé de N-[1-(3-carboxy-1-oxo)-propyl-2-pipéridyl]méthyl)-2, 5-bis(2,2,2-trifluoréthoxy)-benzamide et de fluorescéine-thiocarbamyle-éthylènediamine.

18. Anticorps préparé sous l'influence d'un immunogène suivant la revendication 16.

19. Composé répondant à la formule:

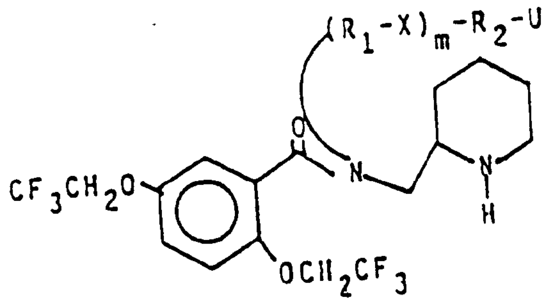

dans laquelle :

m est égal à 0, 1 ou 2; et $R_1$, U et X sont tels que définis dans la revendication 11, $R_2$ est un groupe alkylène de 1 à 12 atomes de carbone, sous les conditions que, si m est égal à 0 et U représente -OH ou -$NHR^{11}$, $R_2$ est alors un groupe alkylène d'au moins 2 atomes de carbone, lié en bout à et séparant U et l'azote d'amide; si m est égal à 1 ou 2 et U représente -OH ou -$NHR^{11}$, $R_2$ est alors un groupe alkylène d'au moins 2 atomes de carbone, lié en bout à et séparant U et le fragment X le plus proche de U; et si m est égal à 2, $R_2$ ne comporte alors pas plus de 6 atomes de carbone.

20. Composé suivant la revendication 19, dans la formule duquel U représente -COOH.

21. Le composé N-(3-carboxypropyl)-N-(2-pipéridylméthyl)-2,5-bis-(2,2,2-trifluoréthoxy)benzamide, suivant la revendication 19.

22. Immunogène comprenant un composé suivant la revendication 19, dans la formule duquel U est différent de -CN, lié par l'intermédiaire de U à un polymère antigénique.

23. Anticorps élaboré sous l'influence d'un immunoène suivant la revendication 22.